# EUROPEAN PATENT APPLICATION

(11) **EP 3 000 439 A1**
(43) Date of publication of application: **30.03.2016**
(21) Application number: 14800737.0
(22) Date of filing: 19.05.2014
(51) Int. Cl.: A61F 2/28, G06T 17/00

(54) **MAXILLOMANDIBULAR PROSTHESIS AND PRODUCTION METHOD**

(30) Priority: 21.05.2013 ES 201330734 P
(71) Applicant: Fundación para la Investigación Hospital Clínico Universitario de Valencia, 46010 Valencia (ES); Asociación de Investigación de La Industria Metal Mecánica, Afines y Conexas, 46988 Paterna (ES); Instituto de Biomecanica de Valencia, 46022 Valencia (ES)
(72) Inventor: PUCHE TORRES, Miguel, E-46010 Valencia (ES); PETROVIC, Vojislav, E-46988 Paterna (ES); GÓMEZ HERRERO, Juan Antonio, E-46022 Valencia (ES); BLASCO PUCHADES, José Ramón, E-46988 Paterna (ES); PORTOLÉS GRIÑAN, Luis, E-46988 Paterna (ES); FERRIS OÑATE, Javier, E-46022 Valencia (ES); ATIENZA VICENTE, Carlos M., E-46022 Valencia (ES)
(74) Representative: Polo Montañes, Carlos
(86) International application number: PCT/ES2014/070409
(87) International publication number: WO 2014/188036

(57) **Abstract**

The invention relates to a prosthesis of maxillomandibular bone defects, of the type used to replace defective bones, and to a method for the production thereof, said prosthesis comprising at least one body (1) with a morphology adapted to the anatomy of the patient, generated from a computerized tomography image of same, where the ends (1a, 1b) thereof have openings (2) for fixation to the ends of the bones of the patient, characterized in that support areas (3) are located between said ends (1a, 1b), on the bone sections, said support areas (3) having a porous geometry containing a second solid, smooth and intermediate area (4) between said support areas (3).

## Description

The present invention relates to a maxillomandibular prosthesis the object of which is to replace a maxillomandibular bone defect. A second object of the invention relates to the production method for producing said prosthesis. Another object of the present invention is a prosthesis having structural features of porosity at the ends thereof, at the level of the bone-prosthesis interface favoring osseointegration as well as greater mechanical strength.

### State of the Art

Bone reconstruction bone at the maxillomandibular level is necessary today due to the existence of defects maxillomandibular having various etiologies. A multidisciplinary approach is required for treatment thereof given the significant impact on the individual's functionality and appearance.

The most common indications for maxillary and/or mandibular reconstruction after the loss of a segment of bone are: partial maxillary and/or mandibular resection due to neoplasia that spreads to the bone, odontogenic neoplasias, losses of bone due to trauma, osteomyelitis causing bone destruction, processes of bisphosphonate-induced mandibular bone osteoradionecrosis and/or necrosis.

Likewise, there are many therapeutic options for said reconstruction: primary closure, allografts, autologous grafts and local and/or microsurgical tissue transfer. Fortunately, reconstructive surgical techniques of the head and neck have evolved, as well as the technology of manufacturing reconstruction plates, such that the functional and aesthetic restitution of a patient suffering from a maxillary and/or mandibular defect is possible.

However, it is not always possible to resort to conventional techniques of local or microsurgical tissue transfer, either because there are no donor areas or due to functional contraindication of the patient for a given complexity of microsurgical reconstructive techniques. Other times, the previously placed reconstruction plates suffer, as a result of the load to which they are subjected, a fracture due to a microtrauma, making it necessary to undertake new solutions for the bone defect.

As a result, the ideal ends sought in any maxillary and/or mandibular reconstruction are essentially the following:
a) Restoring maxillary and/or mandibular continuity and function.
b) Recovering the maxillomandibular occlusal relationships.
c) Allowing future dental restoration and mastication, with an aesthetic appearance of symmetry, height and curvature of the mandible and/or of the maxilla.

The jaw movements made in basic mastication, speaking, deglutition and/or yawning functions, mean that the jaw is exposed to significant stress resulting from opposing forces, since compressive and tension forces are generated between the upper and lower jaw, and torsional forces are generated in the inter-canine anterior mandibular region. The region where tension forces are equal to compressive forces is referred to as the ideal line, and it is a plane located at the base of the alveolar process just below the root apices of the teeth. Therefore, it is vitally important to know the distribution of those forces for suitable placement of the plates and screws during reconstruction.

Bone defects can be classified in a number of ways (partial defects, hemimandibular defects, hemimaxillary defects, total defects, body defects, symphysis defects, etc.). In addition, many bone defects entail associated defects in soft tissue, which also must be considered when considering reconstruction.

Various systems have been developed for replacing the removed maxillary and/or mandibular portion, such that plastic (acrylic) or metal, for example titanium, prosthesis systems have been designed. The following stand out among such systems:
- Rigid fixation systems are considered the basic maxillary and/or mandibular reconstruction methods. These systems include a reconstruction plate with or without bone grafting. Use of the reconstruction plate is indicated for bridging defects, but it is necessary to associate a bone graft with it which, once regenerated, favors biomechanical stability.
- Titanium reconstruction plate systems have been the cornerstone of reconstruction in recent years. These prostheses are fixed to the remaining ends generally using screwed-in systems. There are systems in which the titanium reconstruction plate is pre-formed, bending it such that its new shape resembles the shape of the remaining bone and bridges the defect created during exeresis.

Other techniques are based on using tubular- or channel-shaped titanium mesh that is secured to the bone and allowing housing grafted bone. These meshes are modeled according to the defect after excision of a tumor and are fixed together with non-vascularized bone grafting. Titanium meshes have a high failure rate.

All these systems, which have been used for years, are not without problems and do show deficiencies.

One of the drawbacks is the need for modeling in the operating room, using up surgery time, usually between 30 and 60 minutes, due to the difficulty of adopting the systems to the anatomy of a patient.

Another drawback is the handling of the plate, which can potentially cause fatigue for the metal due to inappropriate handling during modeling. Although these systems entailed a precise system in the challenge of jaw reconstruction, it is also true that they present complications, such as wound dehiscence with plate exposure, plate fracture, and loss of fixation at osteosynthesis screw fixation points. These complications occur in 30-40% of cases.

Therefore, optimization of prosthetic designs by means of CAD-CAE techniques, which have become widespread in recent years, is currently presented as the only technique possible for achieving better designs. Current techniques for additive manufacturing (also known as additive layer manufacturing, rapid manufacturing and 3D printing), together with greater flexibility of integrated production lines, allow responding to this demand for short (even single) runs in shorter times, individualized manufacture being possible today. The development of active ingredients and new processed materials in additive manufacturing techniques has allowed for the manufacture of a completely functional medical device at both the surgical model level and the prosthesis and implant level. This technology enables improving fixation elements and techniques. Furthermore, it reduces costs due to the reduction in times in each of the pre-operatory and post-operatory phases. The surgeon can test the specific surgical technique in models before the intervention.

The applicant knows of the existence of a number of documents in the state of the art, such as patent document US5741215, disclosing a method of manufacture by means of the stereolithographic modeling technique with respect to an implant. The materials used can vary: they can be inorganic of ceramic materials, hydroxyapatite, biocompatible metals such as titanium, and polymeric organic materials such as acrylic polymers, polyesters, polyolefins, polyurethanes, silicones, Teflon, etc., and furthermore it can have solid and other porous parts.

Another example is patent document US2006019216, disclosing a method of manufacturing a jaw implant from a rapid prototyping model consisting of stereolithography, selective laser sintering, deposition modeling, 3D printing, etc. Titanium as well as porous parts in the implant are described as the materials.

Patent document US2003065259 discloses a method of fabricating by means of technique of stereolithography of a surgical splint to receive a patient's dentition as well as part of the upper jaw, lower jaw and part of the cranial bone.

Patent document US2009042167 discloses a prosthesis installed in the jawbone for the placement of at least one tooth. This prosthesis was manufactured by means of the 3D printing or stereolithography (SLA) technique, the material used being titanium. Furthermore, the document describes a prosthesis having porous characteristics in one of the steps of the manufacture thereof.

Patent document US2005153257 discloses an additive (3D) manufacturing method (3D) from a bite registration impression of the patient, which allows obtaining alignment occlusal of the jaw.

Patent document RU2007133573 discloses manufacturing a temporary jaw implant using the stereolithography technique. It describes the use of an osteoplastic material for filling in gaps, as well as a cemented temporary dental bridge.

### Disclosure of the Invention

The present invention describes a maxillomandibular prosthesis, of the type of the used to replace defective maxillary and/or mandibular bones, comprising at least one body with a morphology adapted to the anatomy of the patient generated from a image CT (computerized tomography) of same, and where the ends thereof have openings for fixation to the bone of the patient, preferably by means of screws, such that they encircle, are fitted and/or coupled to the maxilla and/or mandible of the patient, and characterized in that adjacent to said ends there are support areas with the bone sections, said support areas having a porous geometry that allows for bone growth; and where it finally has a solid, smooth and intermediate area causing ligation with soft tissue.

The production method for producing the prosthesis herein proposed first starts from a three-dimensional model of the part to be replaced. Then, from said model, the body is manufactured by means of biocompatible materials, such as a titanium alloy, for example, in powder format, by additive layering by means of supplying energy.

As a result, the capacity for obtaining the geometry of the implant customized for each patient is attained, since it is possible to generate a customized CAD of the implant to be manufactured by means of a 3D scanner. The implant is manufactured directly from this CAD, by means of additive technologies, optimizing the weight of the implant, without losing its resistive capacity, saving in material used for the manufacture thereof. Additive technologies in metal are described, for example, in standard ASTM F2792 - 12a "*Standard Terminology for Additive Manufacturing Technologies".*

As a result, the problem of implant temperature sensitivity is solved since the influence of temperature when manufacturing three-dimensional structures is reduced, since the implants will have lower material density material with respect to volume.

Customizing the prosthesis greatly favors better adaptation thereof to the bone, preventing allowances due to sizing.

Linear or progressive osteoinductive structures can therefore be created for optimal bone growth. By working with a specific CAD, it is possible to generate controlled structures with defined pore sizes, being able to generate structures aiming for better bone integration. Furthermore, areas with greater porosity favoring greater bone growth, and other areas with more reduced porosity limiting bone growth can be generated in the CAD design. In addition to control in the distribution and size of the pore by areas, a connection can be generated between them, giving rise to a continuity of cavities favoring osseointegration of the implant. These structures can be manufactured by means of additive manufacturing technologies.

From the 3D CAD and with optimal planning of the manufacture, any prosthesis can be manufactured in a matter of hours using biocompatible material addition technologies. Nevertheless, prosthesis designs can be optimized by removing material where it is not needed to reduce the manufacturing time, and to that end it will be necessary to design or redesign the prosthesis in the belief that this technology, unlike of conventional technologies, allows for greater design freedom and an important savings in material. If the geometry of a prosthesis is redesigned and its volume reduced by half, the manufacturing time could be significantly reduced.

This also results in a shorter surgical intervention time, since a prototype can be generated from a 3D CAD in any other material, where said prototype allows studying the surgical intervention beforehand, evaluating the design of the implant, its adaptation to the body of the patient or any other factor of interest for the surgeon. This entails an evident reduction in intervention time. Manufacturing cutting guides that help the surgeon with precision and speed in performing the mandibular and/or maxillary resection is also allowed.

In turn, this entails a shorter recovery time for the patient because since the can be studied outside the operating room by means of manufacturing a prototype, intervention time is evidently reduced. It is estimated that one more hour of intervention implies another day of recovery for the patient.

The word "comprises" and variants thereof used throughout the description and the claims do not seek to exclude other technical features, additions, components or steps. For the persons skilled in the art, other objects, advantages and features of the invention will be inferred in part from the description and in part from putting the invention into practice. The following examples and drawings are provided by way of illustration, and they do not seek to restrict the present invention. Furthermore, the present invention covers all the possible combinations of particular and preferred embodiments indicated herein.

### Brief Description of the Drawings

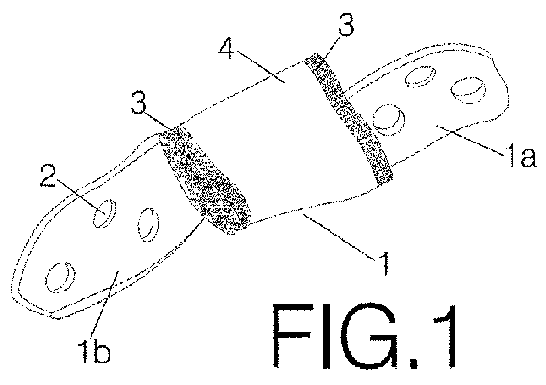

Figure 1 shows a first view of a practical embodiment of the maxillomandibular prosthesis, object of the present invention.

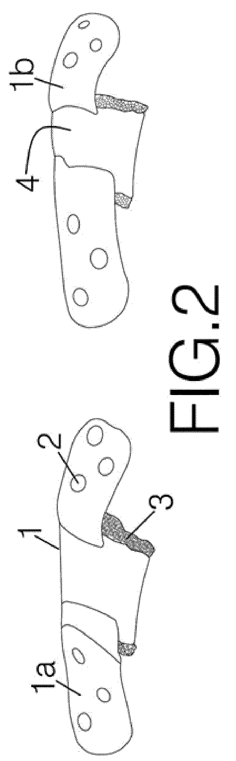

Figure 2 shows a second view of a practical embodiment of the maxillomandibular prosthesis, object of the present invention.

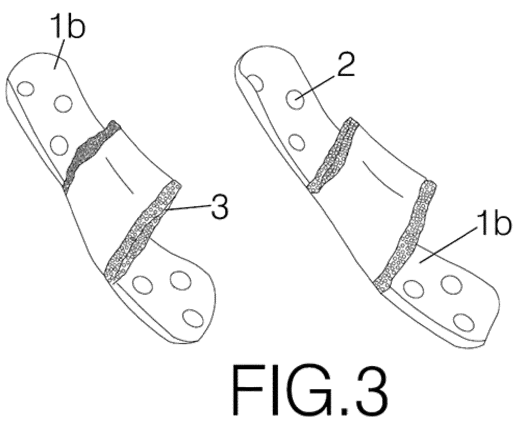

Figure 3 shows a third view of a practical embodiment of the maxillomandibular prosthesis, object of the present invention.

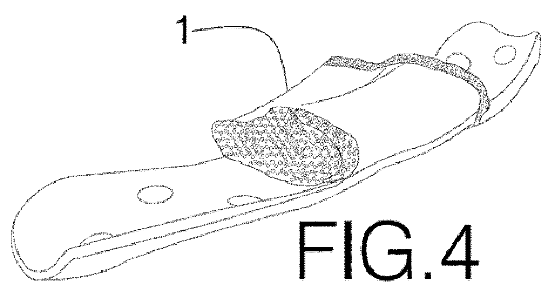

Figure 4 shows a fourth view of a practical embodiment of the maxillomandibular prosthesis, object of the present invention.

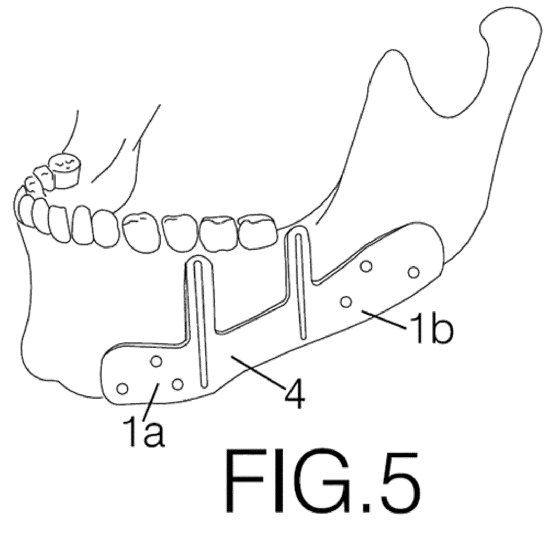

Figure 5 shows a first view of the guide for performing bone resection prior to implanting the maxillomandibular prosthesis in a mandible.

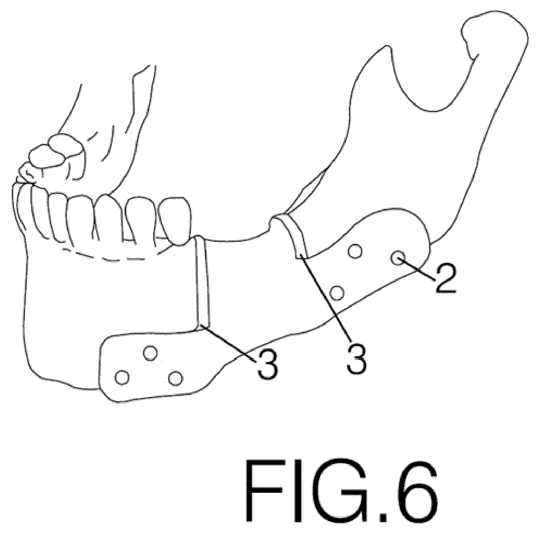

Figure 6 shows a second view of the maxillomandibular prosthesis in a mandible.

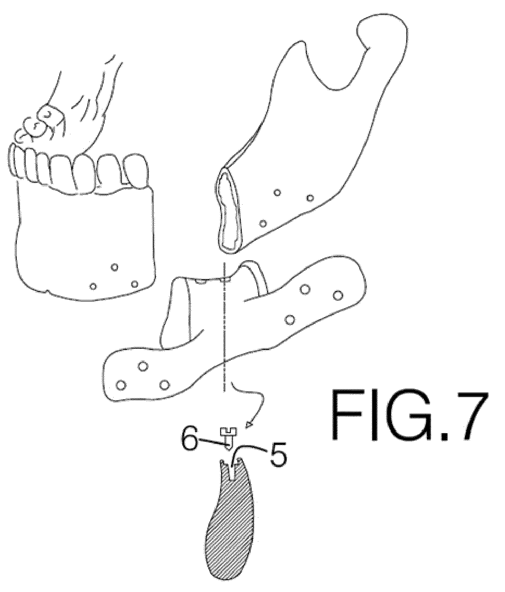

Figure 7 shows a practical embodiment of the maxillomandibular prosthesis, in which it has a universal connection (5) for dental attachments (6).

### Preferred Embodiment of the Invention

The attached drawings show a particular, non-limiting embodiment of the invention. More specifically, the maxillomandibular prosthesis, object of the present invention, is essentially characterized by comprising at least one body (1) with a morphology adapted to the anatomy of the patient generated from a image CT of same, and where the ends (1a, 1b) thereof have openings (2) for fixation to the bone of the patient, preferably by means of screws, such that encircle, are fitted and/or coupled to the mandible and/or maxilla of the patient.

Two support areas (3) with the bone sections are located adjacently, said support areas (3) having a porous geometry that allows for bone growth and the anchoring of the replacement part to the bone with limited depth, and the cross-section of which has the same outline as the sectional area of the bone on which is it supported.

Finally, the prosthesis has a solid intermediate area (4) providing mechanical rigidity and characterized by having a morphology identical to that of the area of the bone that is replaced generated from a three-dimensional symmetrical model of the undamaged area of the bone.

As a result of its special rough morphology, the body (1) favors the osseointegration process in areas where the bone must be anchored to the replacement part, and where, as a result of the smooth surface thereof in contact with soft tissue, produces ligation between both.

The porous area has a distribution, size, and pore connection suitable for the growth and anchoring of the bone and for the extraction of the powder that did not melt during the production method.

In another non-limiting, practical embodiment, the body (1) will be divided into two parts that will be fitted together by means of a housing and a recess provided for such purpose and they will be fixed together by means of a set screw, where the porous area will only be present in one of said parts, and the smooth part will have openings (2) for the fixation thereof in the patient or recipient.

In a non-limiting, practical embodiment, the body (1) will have on the occlusal or upper surface thereof at least one universal connection (5) for housing dental attachments (6), preferably with an inner threading.

The production method for producing the prosthesis herein proposed starts from a three-dimensional model to which the porous area of the prosthesis is added by computer with complete control with respect to distribution, size and connection of the pore, and from said digitally modified model, the body (1) is produced by means of using biocompatible materials (such as titanium alloy in powder format, for example) by additive layering, by means of supplying energy (such as energy from an electron beam and/or laser, for example).

In a second non-limiting, practical embodiment, the production method for producing the prosthesis herein proposed starts from the digitally modified model for producing the body (1), by means of using meltable or burnable materials, by additive layering since a physical model made of a material would be obtained which, by applying a temperature curve thereto in a furnace for a certain time period, melts (meltable material, e.g. wax) or burns (burnable material, e.g. resin). Once this model is made by layering, it can be used to obtain a body (1) made of a biocompatible metal.

To that end, a ceramic mold would be manufactured and the cavity thereof would be the negative of the model produced. When the material of the model is placed in the furnace, it melts or burns, leaving an empty space. A molten metal can then be introduced in that empty space by gravity, for example, with a slight negative pressure or excessive pressure, which helps to fill the mold. The final metal part is taken out when the mold is broken. The process must be repeated every time a part is to be taken out.

A third practical embodiment for use in cases of using of biocompatible polymeric materials would be based on making mold using silicone (from the model obtained by layering) which would serve for casting (by means of a vacuum or the like) two liquid thermosetting materials, chemically reacting with one other to go from the liquid to solid state, the silicone mold is finally cut, obtaining the final part. In this specific case, the mold could be used to produce several parts.

A final production possibility could be to use machining methods in which once the digitally modified model has been obtained, numerical control machines are used to obtain the final part.

## Claims

1. A maxillomandibular prosthesis, of the type of the used to replace defective bones, comprising at least one body (1) with a morphology adapted to the anatomy of the patient generated from a computerized tomography image of same, and where the ends (1a, 1b) thereof have openings (2) for fixation to the ends of the bones of the patient; where said prosthesis is **characterized in that** support areas (3) are located between said ends (1a, 1b), on the bone sections, where said support areas (3) have a porous geometry containing a second solid, smooth and intermediate area (4) between said support areas (3).

2. The prosthesis according to claim 1, wherein the body (1) is divided into two parts that fit with one another, by means of a housing and a recess and that are fixed by means of a set screw.

3. The prosthesis according to claim 2, wherein the support area (3) is located in one of the parts of the body (1) that fit together.

4. The prosthesis according to any of claims 1 to 3, wherein the second solid and smooth area (4) is located in one of the parts of the body (1) and has openings (2) with the ends of the bones of the patient.

5. The prosthesis according to any of claims 1 to 4 wherein the body (1) has on its occlusal or upper surface at least one connection (5) for housing dental attachments (6).

6. A production method for producing the prosthesis according to any of claims 1 to 5, comprising the steps of obtaining a three-dimensional model of the maxillomandibular bone structure to be replaced, to which a porous area of the prosthesis is added by computer controlling the distribution, size and connection of the pore; and wherein there is established from said modified model a step of manufacturing the body (1) by means of biocompatible materials, preferably in powder format, by additive layering by means of supplying energy.

7. The production method for producing the prosthesis according to claim 6, where the step of manufacturing the body (1) comprises the step of obtaining a physical model from the modified model by computer, by additive layering, obtaining a physical model made of a material to which a temperature curve is applied in a furnace for a certain time period, said material melting or burning, obtaining a mold that renders a space for later introducing a molten biocompatible material, filling the mold, and where said mold is finally broken, obtaining the final metal part.

8. The production method for producing the prosthesis according to claim 6, wherein the step of manufacturing the body (1) comprises the step of obtaining a physical model from the modified model by computer, by additive layering, obtaining a silicone mold wherein two liquid thermosetting materials are introduced, chemically reacting with one another, and wherein said mold is finally cut to obtain the final part.
